# EUROPEAN PATENT APPLICATION

(11) **EP 2 154 152 A1**
(43) Date of publication of application: **17.02.2010**
(21) Application number: 08014233.4
(22) Date of filing: 08.08.2008
(51) Int. Cl.: C07K 14/435

(54) **Use of Conkunitzin-S1 for the modulation of glucose-induced insulin secretion**

(71) Applicant: Max-Planck-Gesellschaft zur Förderung der Wissenschaften e.V., 80539 München (DE); Universität zu Lübeck, 23538 Lübeck (DE)
(72) Inventor: The designation of the inventor has not yet been filed
(74) Representative: Vossius & Partner

(57) **Abstract**

The present invention relates to a (poly)peptide or a peptidomimetic thereof having the biological activity of Conkunitzin-S1, wherein said (poly)peptide is selected from (a) a polypeptide comprising or having the amino acid sequence of SEQ ID NO: 1; (b) a polypeptide having at least 85% sequence identity to SEQ ID NO:1; or (c) a fragment of a) or b); wherein said (poly)peptide or peptidomimetic specifically modulates the activity of a channel having the activity of a Kv1.7 containing channel, for the treatment or prevention of metabolic diseases or conditions, or secondary diseases or conditions related to said metabolic diseases or conditions. The present invention furthermore relates to a method of screening for (poly)peptides derived from Conkunitzin-S1 suitable for specifically modulating the activity of a channel having the activity of a Kv1.7 containing channel, comprising: (a) altering the amino acid sequence of Conkunitzin-S1 represented by SEQ ID NO: 1 by deleting and/or inserting and/or replacing at least one amino acid; and (b) determining the modulatory effect of the (poly)peptide obtained in step (a) (i) on a channel having the activity of a Kv1.7 containing channel and (ii) on channels, preferably potassium channels, not having the activity of a Kv1.7 containing channel, which are optionally expressed on the same cell as the channel having the activity of a Kv1.7 containing channel; wherein a modulatory effect of the (poly)peptide determined in step (i) that is at least 50% of the modulatory effect of Conkunitzin-S1 indicates that the (poly)peptide is suitable for modulating the activity of said channel having the activity of a Kv1.7 containing channel; and wherein the determination of essentially no modulatory effect in step (ii) indicates that the (poly)peptide is specifically modulating the activity of channels having the activity of a Kv1.7 containing channel.

## Description

The present invention relates to a (poly)peptide or a peptidomimetic thereof having the biological activity of Conkunitzin-S1, wherein said (poly)peptide is selected from (a) a polypeptide comprising or having the amino acid sequence of SEQ ID NO: 1; (b) a polypeptide having at least 85% sequence identity to SEQ ID NO:1; or (c) a fragment of a) or b); wherein said (poly)peptide or peptidomimetic specifically modulates the activity of a channel having the activity of a Kv1.7 containing channel, for the treatment or prevention of metabolic diseases or conditions, or secondary diseases or conditions related to said metabolic diseases or conditions. The present invention furthermore relates to a method of screening for (poly)peptides derived from Conkunitzin-S1 suitable for specifically modulating the activity of a channel having the activity of a Kv1.7 containing channel, comprising: (a) altering the amino acid sequence of Conkunitzin-S1 represented by SEQ ID NO: 1 by deleting and/or inserting and/or replacing at least one amino acid; and (b) determining the modulatory effect of the (poly)peptide obtained in step (a) (i) on a channel having the activity of a Kv1.7 containing channel and (ii) on channels, preferably potassium channels, not having the activity of a Kv1.7 containing channel, which are optionally expressed on the same cell as the channel having the activity of a Kv1.7 containing channel; wherein a modulatory effect of the (poly)peptide determined in step (i) that is at least 50% of the modulatory effect of Conkunitzin-S1 indicates that the (poly)peptide is suitable for modulating the activity of said channel having the activity of a Kv1.7 containing channel; and wherein the determination of essentially no modulatory effect in step (ii) indicates that the (poly)peptide is specifically modulating the activity of channels having the activity of a Kv1.7 containing channel.

In this specification, a number of documents including patent applications and manufacturer's manuals is cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Potassium channels comprise a large and diverse group of proteins that maintain the cellular membrane potential and are thus fundamental in biological function. The channels can be broadly subdivided into different groups including voltage-gated K⁺ channels, Ca²⁺ activated K⁺ channels and ATP-sensitive K⁺ channels. Abnormal flow of potassium ions through these channels is associated with a number of disorders.

Voltage-gated (Kv) potassium (K⁺) channels are transmembrane channels specific for potassium and sensitive to voltage changes in the cell's membrane potential. They play a crucial role during action potentials in returning the depolarized cell to a resting state. Voltage-gated K⁺ channels of vertebrates are tetramers of four subunits (alpha subunits) arranged as a ring, each contributing to the wall of the trans-membrane K⁺ pore. Each subunit is comprised of six membrane spanning hydrophobic α-helical sequences. The selectivity of voltage-gated K⁺ channels for K⁺ over other cations such as Na⁺ is mediated by a selectivity filter at the narrowest part of the transmembrane pore.

Attempts continue to relate the structure of mammalian voltage-gated K⁺ channels to their ability to respond to the voltage that exists across the membrane. Specific domains of the channel subunits have been identified that are important for voltage-sensing and converting between the open and closed conformations of the channel. There are at least two closed conformations; in one, the channel can open if the membrane potential becomes positive inside. Alternatively, voltage-gated K⁺ channels inactivate after opening, entering a distinctive, second closed conformation. In this inactivated conformation, the channel cannot open, even if the transmembrane voltage is favorable. This inactivation is mediated by a domain at one end of the K⁺ channel protein that can transiently plug the inner opening of the pore, thus preventing ion movement through the channel.

Voltage-gated potassium channels may further comprise beta subunits which are auxiliary proteins which associate with alpha subunits in a α₄β₄ stoichiometry. These subunits do not conduct current on their own but rather modulate the activity of Kᵥ channels.

The glucose-induced insulin secretion of pancreatic β-cells strongly depends on membrane potential changes of the β-cell. Although numerous voltage-gated potassium channels (Kv) have been identified in pancreatic islets, their role in electrical excitability and glucose regulation of insulin secretion is poorly understood. US patent 5,559,009 identifies a Kv channel subunit termed Kv1.7 which is expressed in pancreatic β-cells and shown to form functional potassium channels. However, the proportion of the potassium flow of channels comprising Kv1.7 is only a minor component of the total potassium current measured in β-cells and is thus believed not to play a major role in the regulation of membrane potential changes and insulin release of β-cells.

Non-insulin-dependent diabetes mellitus (NIDDM) or diabetes mellitus type 2 is a metabolic disorder that is primarily characterized by insulin resistance, relative insulin deficiency and hyperglycemia. It is often managed by engaging in exercise and modifying one's diet. It is rapidly increasing in the developed world, and there is some evidence that this pattern will be followed in much of the rest of the world in the coming years. Complex and multifactorial metabolic changes very often lead to damage and function impairment of many organs, most importantly the cardiovascular system. This leads to substantially increased morbidity and mortality. Diabetes type 2 is often caused by as well as leads to other diseases and conditions such as metabolic syndrome, hypertension, diabetic retinopathy, cardiac infarction, peripheral vascular disease, stroke or central obesity. All these diseases and conditions form a network of interrelated diseases which determine one another. Drugs which target diabetes type 2 may thus also relieve secondary diseases of diabetes type 2. Depending on the mechanism of action, they may, however, also be able to target diseases which cause diabetes mellitus type 2.

Several drugs are by now available for the treatment of diabetes mellitus type 2. The most important drug presently in use is the biguanide metformin which acts primarily by reducing blood glucose from glycogen stores in the liver as well as provoking an increase in cellular uptake of glucose in body tissues. Both historically and currently commonly used are substances belonging to the sulfonylurea group, of which several members (including glibenclamide and gliclazide) are widely used; these substances increase glucose stimulated insulin secretion by the pancreas. More recently developed drug classes include: Thiazolidinediones (TZDs) which increase tissue insulin response, α-glucosidase inhibitors which interfere with absorption of some nutrients, meglitinides which stimulate insulin release quickly or peptide analogues which work in a variety of ways. A major disadvantage of most of these drugs is that they severely reduce glucose levels in the blood below the physiological level and thereby induce hypoglycemia.

Conotoxins are a class of peptides of generally up to 30 amino acids in length produced by molluscs of the genus *Conus* which were identified to exert several functions. α-, µ- and ω-conotoxins target acetylcholine receptors, muscle sodium channels and neuronal calcium channels. Recently, a further group of conotoxins called conkunitzins has been identified. Conkunitzins are suggested to block potassium channels (WO2006/098764). One prominent member of this group is Conkunitzin-S1, which is unusually long with 60 amino acids and , which was shown to block *Drosophila* melanogaster *Shaker* potassium channels (Bayrhuber et al., 2005).

Although several drugs are by now available for the treatment of diabetes mellitus type 2, it is still desirable to develop further drugs with more advantageous properties, e.g. drugs not inducing hypoglycemia. The solution to this problem is provided by the embodiments characterized in the claims.

Accordingly, the present invention relates to a (poly)peptide or peptidomimetic thereof having the biological activity of Conkunitzin-S1, wherein said (poly)peptide is selected from (a) a polypeptide comprising or having the amino acid sequence of SEQ ID NO: 1; (b) a polypeptide having at least 85% sequence identity to SEQ ID NO: 1; or (c) a fragment of (a) or (b), wherein said (poly)peptide or peptidomimetic specifically modulates the activity of a channel having the activity of a Kv1.7 containing channel, for the treatment or prevention of metabolic diseases or conditions, or secondary diseases or conditions related to said metabolic diseases or conditions.

The invention also relates to a method of treating or preventing metabolic diseases or conditions, or secondary diseases or conditions related to said metabolic diseases or conditions comprising administering a pharmaceutically effective amount of a (poly)peptide or a peptidomimetic thereof having the biological activity of Conkunitzin-S1, wherein said (poly)peptide is selected from (a) a polypeptide comprising or having the amino acid sequence of SEQ ID NO: 1; (b) a polypeptide having at least 85% sequence identity to SEQ ID NO: 1; or (c) a fragment of (a) or (b), , wherein said (poly)peptide or peptidomimetic specifically modulates the activity of a channel having the activity of a Kv1.7 containing channel, to a subject in need thereof.

Conkunitzin S1 is a 60-residue neurotoxin from the venom of the cone snail *Conus striatus* that blocks *Shaker* potassium channels found in *Drosophila.* In the context of the present invention, Conkunitzin-S1 as represented by the amino acid sequence of SEQ ID NO: 1 (Bayerhuber et al., 2005) was found to specifically interact with mammalian voltage-gated potassium channels comprising Kv1.7. The Conkunitzin-S1 sequence is homologous to that of Kunitz-type proteins, small, basic protein modules having three highly conserved cysteine bridges, but contains only two out of said three cysteine bridges. The only post-translational modification known so far is amidation of the C-terminal carboxylic acid. As this modification is not essential for the activity of Conkunitzin-S1, recombinantly or synthetically produced Conkunitzin-S1 not having said modification essentially exerts the same biological activity.

The term "(poly)peptide" as used herein describes a group of molecules which comprises the group of peptides, consisting of up to 30 amino acids, as well as the group of polypeptides, also termed proteins, consisting of more than 30 amino acids. Accordingly, Conkunitzin as represented by SEQ ID NO: 1 falls under the term "polypeptide". The above recited fragments, on the other hand, may fall under the term "peptide".

(Poly)peptides may further form oligomers consisting of at least two identical or different molecules. The corresponding higher order structures of such multimers are, correspondingly, termed homo- or heterodimers, homo- or heterotrimers etc. The term "(poly)peptide" also refers to naturally modified (poly)peptides where the modification is effected e.g. by glycosylation, acetylation, phosphorylation, amidation and similar modifications which are well known in the art.

The (poly)peptide of the invention can be produced in various ways, including recombinantly, synthetically or semisynthetically.

A large number of suitable methods exist in the art to produce recombinant (poly)peptides in an appropriate host. The host may be unicellular or a multicellular organism. If the host is unicellular, it may be a unicellular organism such as a prokaryote (e.g. E. coli or B. subtilis) or a yeast cell. Unicellular hosts also comprise cells derived from multicellular organisms, such as mammalian cells (e.g. HEK 293, CHO cells), cells of a multicellular fungusor insect cells (SF9 or H5). The person skilled in the art can revert to a variety of culture conditions to express the (poly)peptide as defined above from a nucleic acid transferred into said host. Conveniently, the produced (poly)peptide is harvested from the culture medium, lysates of the cultured cells or from isolated (biological) membranes by established techniques. In the case of a multicellular organism as a host comprising multiple cells carrying a nucleic acid encoding the (poly)peptide of the invention, a fraction of these cells may serve as source for the (poly)peptide of the invention, for example said fraction may be the harvestable part of a plant.

A preferred method involves the synthesis of nucleic acid sequences encoding the (poly)peptide according to the invention by PCR and its insertion into an expression vector. Subsequently, a suitable host may be transfected or transformed with the expression vector. Thereafter, in the case that the host is a cell, the host is cultured to produce the desired (poly)peptide, which is isolated and purified.

A typical mammalian expression vector contains the promoter element, which mediates the initiation of transcription of mRNA, the protein coding sequence, and signals required for the termination of transcription and polyadenylation of the transcript. Moreover, elements such as origin of replication or drug resistance gene regulators (as part of an inducible promoter) may also be included. Additional elements might include enhancers, Kozak sequences and intervening sequences flanked by donor and acceptor sites for RNA splicing. Highly efficient transcription can be achieved with the early and late promoters from SV40, the long terminal repeats (LTRs) from retroviruses, e.g., RSV, HTLVI, HIVI, and the early promoter of the cytomegalovirus (CMV), chicken beta-actin promoter, CAG-promoter (a combination of chicken beta-actin promoter and cytomegalovirus immediate-early enhancer), the gai10 promoter, human elongation factor 1α-promoter, human actin promoter, CMV enhancer, CaM-kinase promoter, the Autographa californica multiple nuclear polyhedrosis virus (AcMNPV) polyhedral promoter or a globin intron in mammalian and other animal cells. The *AOX1* or *GAL1* promoters can be used in yeast. Besides elements which are responsible for the initiation of transcription, such regulatory elements may also comprise transcription termination signals, such as the SV40-poly-A site or the tk-poly-A site or the SV40, lacZ and AcMNPV polyhedral polyadenylation signals, downstream of the polynucleotide. The co-transfection with a selectable marker such as dhfr, gpt, neomycin, hygromycin allows the identification and isolation of the transfected cells. The dhfr (dihydrofolate reductase) marker is useful to develop cell lines that carry several hundred or even several thousand copies of the gene of interest. Another useful selection marker is the enzyme glutamine synthase (GS) (Murphy et al. 1991; Bebbington et al. 1992). Using these markers, the mammalian cells are grown in selective medium and the cells with the highest resistance are selected.

Possible regulatory elements permitting expression in prokaryotic host cells comprise, e.g., the lac promoter, which can be induced using the lactose analogue isopropylthio-b-D-galactoside (IPTG), *trp* or *tac* promoter, the lacUV5 or the trp promotor in E. *coli.* Selectable markers for prokaryotic cells include tetracycline, kanamycin or ampicillin resistance genes for culturing in E. *coli* and other bacteria.

Suitable prokaryotic hosts comprise e.g. bacteria of the species *Escherichia, Streptomyces, Salmonella* or *Bacillus.* Suitable eukaryotic host cells are e.g. yeasts such as *Saccharomyces cerevisiae* or *Pichia pastoris.* Insect cells suitable for expression are e.g. *Drosophila* S2 or *Spodoptera* Sf9 cells.

Mammalian host cells that could be used include, human Hela, HEK293, H9 and Jurkat cells, mouse NIH3T3 and C127 cells, COS 1, COS 7 and CV1, quail QC1-3 cells, mouse L cells, Bowes melanoma cells and Chinese hamster ovary (CHO) cells.

Appropriate culture media and conditions for the above-described host cells are known in the art. For example, suitable conditions for culturing bacteria are growing them under aeration in Luria Bertani (LB) medium. To increase the yield and the solubility of the expression product, the medium can be buffered or supplemented with suitable additives known to enhance or facilitate both. *E. coli* can be cultured from 4 to about 37 °C, the exact temperature or sequence of temperatures depends on the molecule to be overexpressed. In general, the skilled person is also aware that these conditions may have to be adapted to the needs of the host and the requirements of the (poly)peptide expressed. In case an inducible promoter controls the nucleic acid encoding the (poly)peptide according to the invention in the vector present in the host cell, expression of the (poly)peptide can be induced by addition of an appropriate inducing agent. Suitable expression protocols and strategies are known to the skilled person.

Depending on the cell type and its specific requirements, mammalian cell culture can e.g. be carried out in RPMI or DMEM medium containing 10% (v/v) FCS, 2mM L-glutamine and 100 U/ml penicillin/streptomycin. The cells can be kept at 37 °C in a 5% CO₂, water saturated atmosphere.

Suitable media for insect cell culture is e.g. TNM + 10% FCS or SF900 medium. Insect cells are usually grown at 27 °C as adhesion or suspension culture.

Suitable expression protocols for eukaryotic cells are well known to the skilled person and can be retrieved e.g. from Sambrook, 2001.

An alternative method for producing the (poly)peptide according to the invention is in vitro translation of mRNA. Suitable cell-free expression systems for use in accordance with the present invention include rabbit reticulocyte lysate, wheat germ extract, canine pancreatic microsomal membranes, *E. coli* S30 extract, and coupled transcription/translation systems such as the TNT-system (Promega). These systems allow the expression of recombinant (poly)peptides upon the addition of cloning vectors, DNA fragments, or RNA sequences containing coding regions and appropriate promoter elements.

Methods of isolation of the (poly)peptide produced are well-known in the art and comprise, without limitation, method steps such as ion exchange chromatography, gel filtration chromatography (size exclusion chromatography), affinity chromatography, high pressure liquid chromatography (HPLC), reversed phase HPLC, disc gel electrophoresis or immunoprecipitation (see, for example, Sambrook, 2001). Methods specifically adapted for the purification of Conkunitzin-S1 are disclosed in Bayrhuber et al. (2006) and Becker and Terlau (2008). In case Conkunitzin-S1 is expressed in inclusion bodies in bacteria, the pellet may be dissolved in 6M guanidinium hydrochloride containing a reducing agent and subsequently be refolded. After separation of precipitate the (poly)peptide may be further purified using a cation exchange column followed by HPLC. The methods described above for the purification of Conkunitzin-S1 may also be used for or adapted to the purification of the (poly)peptides as defined above.

Due to their limited size, peptides according to the invention can also conveniently be prepared synthetically.

Chemical synthesis of peptides is well known in the art. Solid phase synthesis is commonly used and various commercial synthesizers are available, for example automated synthesizers by Applied Biosystems Inc., Foster City, CA; Beckman; MultiSyntech, Bochum, Germany etc. Solution phase synthetic methods may also be used, although it is less convenient. By using these standard techniques, naturally occurring amino acids may be substituted with unnatural amino acids, particularly D-stereoisomers, and also with amino acids with side chains having different lengths or functionalities. Functional groups for conjugating to small molecules, label moieties, peptides, or proteins or for purposes of forming cyclized peptides may be introduced into the molecule during chemical synthesis. In addition, small molecules and label moieties may be attached during or after the synthetis process. Preferably, introduction of functional groups and conjugation to other molecules minimally affects the structure and function of the subject peptide.

The N-and C-terminus of the (poly)peptide may be derivatized using conventional chemical synthetic methods. The (poly)peptides of the invention may contain an acyl group, such as an acetyl group or an amide group. Methods for amidating (poly)peptides are well-known in the art. Exemplary methods are disclosed in Ray et al. (2002) describing an enzymatic method to convert C-terminal glycin by reaction with peptidylglycine α-amidating monooxygenase (PAM) and in Cottingham et al. (2001) using (poly)peptide-intein fusion proteins.

Methods for acylating, and specifically for acetylating the free amino group at the N-terminus are well known in the art. For the C-terminus, the carboxyl group may be modified by esterification with alcohols or amidated to form-CONH₂ or CONHR. Methods of esterification and amidation are done using well known techniques.

For example, peptide synthesis can be carried out using Nα-9-fluorenylmethoxycarbonyl amino acids and a preloaded trityl resin or an aminomethylated polystyrene resin with a p-carboxytritylalcohol linker. Coupling can be performed in dimethylformamide using N-hydroxybenzotriazole and 2-(1H-benzotriazole-1-yl)-1,1,3,3-tetramethyluronium hexafluorophosphate. Commonly used side chain protecting groups are tert-butyl for D, E and Y; trityl for N, Q, S and T; 2,2,4,6,7-pentamethyldihydroxybenzofruan-5-sulfonyl for R; and butyloxycarbonyl for K. After synthesis, the peptides are deprotected and cleaved from the polymer support by treatment with e.g. 92% trifluoracetic acid/ 4% triethylsilane/ 4% H₂O. The peptides can be precipitated by the addition of tert-butylether/pentane (8:2) and purified by reversed-phase HPLC. The peptides are commonly analysed by matrix-associated laser desorption time-of-flight mass spectrometry.

In addition, the (poly)peptide of the invention may also be produced semisynthetically, for example by a combination of recombinant and synthetic production. In the case that fragments of the (poly)peptide are produced synthetically, the remaining part of the (poly)peptide would have to be produced otherwise, e.g. recombinantly, and then linked to the fragment to form the (poly)peptide of the invention.

Furthermore, the invention encompasses peptidomimetics of the (poly)peptide as defined above. A peptidomimetic is a small protein- or peptide-like chain designed to mimic a (poly)peptide. Peptidomimetics typically arise from modifications of an existing (poly)peptide in order to alter the molecule's properties. For example, they may arise from modifications to change the molecule's stability. These modifications involve changes to the (poly)peptide that will not occur naturally (such as altered backbones and the incorporation of non-natural amino acids), including the replacement of amino acids or peptide bonds by functional analogues. Such functional analogues include all known amino acids other than the 20 gene-encoded amino acids, such as selenocysteine. The use of peptidomimetics as compared to other mimetics have some particular advantages. For instance, their conformationally restrained structure allows to minimize binding to non-target compounds and enhance the activity at the desired targets. Through the addition of hydrophobic residues and/or replacement of amide bonds the transport of peptidomimetics through cellular membranes can be improved. Furthermore peptidomimetics such as isosters, retro-inverso peptides and cyclic peptides are less susceptible to degradation by peptidases and other enzymes.

The term "biological activity of Conkunitzin-S1" in accordance with the present invention refers to the modulatory, i.e. inhibitory or activating, activity of Conkunitzin-S1 on channels consisting of Kv1.7 subunits as well as on Kv1.7 containing channels. In this regard, the term "inhibitory" refers to a reduced potassium current through these channels whereas the term "activating" refers to an increase in the potassium current through said channels.

In the present invention, the (poly)peptide as well as the peptidomimetic according to the invention have the biological activity of Conkunitzin-S1. Whether a (poly)peptide or peptidomimetic according to the invention has an inhibitory or activating activity on a channel having the activity of a Kv1.7 containing channel dependends on the physiological state of the cell comprising said channel or on the physiological state of the channel itself (closed, open or inactivated). This phenomenon is also known as state dependence and has long been known for locally applied anesthetics. State dependence was examined on potassium channels using the conopeptide PVIIA (Terlau et al., 1999). It was found that under the specific conditions used in this work (a specific expression system and a molecularbiologically altered channel), the conopeptide did no longer inhibit the activity of the channel but led to an increase of the potassium current measured. Similar effects were shown under physiologically relevant conditions for other molecules (Jackson and Bean, 2007). There, the authors showed that 4-aminopyridine, a known potassium channel blocker, could under specific conditions also lead to an increase of the potassium current. Accordingly, whereas the present inventors show that Conkunitzin-S1 inhibits heterologously expressed Kv1.7 channels, an activation of channels having the activity of a Kv1.7 containing channel is also conceivable based on the above.

More specifically, where the biological activity of Conkunitzin-S1 in accordance with the present invention is an inhibitory activity, it is defined as an IC₅₀ of between 10 nM and 5 µM on Kv1.7 channels heterologously expressed in cells capable of functionally expressing said channel on their surface. In this regard, functional expression results in a channel having its native structure, folding and activity. In addition, a "cell capable of functionally expressing said channel" is a cell that provides suitable conditions to allow the channel to exert its activity, which is determined by measuring the potassium currents in the cell. These cells do not naturally express Kv1.7 or homologues of Kv1.7. Preferably, other (voltage-gated) potassium channels are also not expressed by these cells.

Examples of cells suitable for heterologous expression are Xenopus oocytes, HEK 293, CHO or COS cells. Heterologous expression is effected by methods well known in the art (e.g. RNA injection for Xenopus oocytes or transient or stable transfection for HEK 293, CHO or COS cells). These methods are also described in the appended examples. The IC₅₀ is measured by the well known "two electrode voltage clamp" (TEVC) method as also described in detail in the appended examples.

In accordance with the present invention, the term "percent (%) sequence identity" describes the number of matches ("hits") of identical amino acids of two or more aligned amino acid sequences as compared to the number of amino acid residues making up the overall length of the template amino acid sequences. In other terms, using an alignment for two or more sequences or subsequences, the percentage of amino acid residues that are the same (e.g., 85%, 90% or 95% identity) may be determined, when the (sub)sequences are compared and aligned for maximum correspondence over a window of comparison, or over a designated region as measured using a sequence comparison algorithm as known in the art, or when manually aligned and visually inspected.

To evaluate the identity level between two nucleotide or protein sequences, they can be aligned electronically using suitable computer programs known in the art. Such programs comprise BLAST (Altschul et al. (1990) J. Mol. Biol. 215, 403), variants thereof such as WU-BLAST (Altschul and Gish (1996) Methods Enzymol. 266, 460), FASTA (Pearson and Lipman (1988) Proc. Natl. Acad. Sci. USA 85, 2444) or implementations of the Smith-Waterman algorithm (SSEARCH, Smith and Waterman (1981) J. Mol. Biol., 147, 195). These programs, in addition to providing a pairwise sequence alignment, also report the sequence identity level (usually in percent identity) and the probability for the occurrence of the alignment by chance (P-value). Programs such as CLUSTALW (Thompson et al. (1994) Nucleic Acids Res. 22, 4673) can be used to align more than two sequences.

Encompassed by the present invention are (poly)peptides having sequences that exhibit at least 85% identity to Conkunitzin-S1 represented by SEQ ID NO:1. Preferably, the identity is between 85 and 98% such as at least 90%, more preferred at least 95%. It is most preferred that the identity is at least 98% to SEQ ID NO: 1. Molecules falling under this definition may be isoforms, homologous molecules from other species, such as orthologs, or mutated sequences from the same species to mention some preferred examples.

Also encompassed by the present invention are fragments of the amino acid sequence of SEQ ID NO: 1 or of an amino acid sequence having at least 85% sequence identity to SEQ ID NO: 1, wherein one or more amino acids have been deleted to arrive at a shorter (poly)peptide.

It is well known in the art that functional (poly)peptides may be cleaved to yield fragments with unaltered or substantially unaltered biological activity. Such cleavage may include the removal of a given number of N- and/or C-terminal amino acids. Additionally or alternatively, a number of internal (non-terminal) amino acids may be removed, provided the obtained (poly)peptide retains the biological activity of Conkunitzin-S1. Said number of amino acids to be removed from the termini and/or internal regions may be one, two, three, four, five, six, seven, eight, nine, ten, 15, 20, 25 or more than 25. Any other number between one and 25 is also deliberately envisaged. In particular, removal of amino acids which preserve sequence and boundaries of any conserved functional domain(s) or subsequences in the sequence of SEQ ID NO: 1 are particularly envisaged. Means and methods for determining such domains are well known in the art and include experimental and bioinformatic means. Experimental means include the systematic generation of deletion mutants and their assessment in assays for the biological activity of Conkunitzin-S1 known in the art and as described in the appended examples. Bioinformatic means include database searches. Suitable databases include protein sequence databases. In this case a multiple sequence alignment of significant hits is indicative of domain boundaries, wherein the domain(s) is/are comprised of the/those subsequences exhibiting an elevated level of sequence conservation as compared to the remainder of the sequence. Further suitable databases include databases of statistical models of conserved protein domains such as Pfam maintained by the Sanger Institute, UK (www.sanger.ac.uk/Software/Pfam).

Accordingly, the fragment as defined above as envisaged in the present invention comprises the pharmacophore of Conkunitzin-S1, i.e. the part of the Conkunitzin-S1 molecule necessary and sufficient to exert the biological activity of Conkunitzin-S1.

A "channel having the activity of a Kv1.7 containing channel" is a voltage-gated potassium channel possessing the same biological activity as a naturally occurring Kv1.7 containing channel, preferably as represented in SEQ ID NO: 2. This biological activity is represented, e.g., by a sensitivity to Conkunitzin-S1. Said channel having the activity of a Kv1.7 containing channel is comprised of four subunits each contributing to the ion channel activities, wherein at least one of the subunits is either Kv1.7 or a derivative thereof. Said derivative of Kv1.7 may comprise an altered amino acid sequence as compared to that of naturally occurring Kv1.7, wherein at least one amino acid is deleted, inserted or replaced/substituted. Any alteration or combination of alterations may be effected as long as the resulting Kv1.7 derivative retains the above properties, e.g. its sensitivity to Conkunitzin-S1, and, in combination with three other subunits, its ability to form a functional voltage-gated potassium channel.

The term "a (poly)peptide or peptidomimetic specifically modulating the activity of a channel having the activity of a Kv1.7 containing channel" in accordance with the present invention denotes the selective modulatory effect of a (poly)peptide or peptidomimetic according to the present invention on a Kv1.7 containing channel, whereas essentially no such effect is observed for channels not containing Kv1.7. For example, if an inhibitory effect on a channel having the activity of a Kv1.7 containing channel is found for a (poly)peptide or peptidomimetic, this (poly)peptide or peptidomimetic is regarded as specifically modulating the activity of said channel if it essentially neither inhibits nor activates channels not having the activity of a Kv1.7 containing channel. In this regard, the term "essentially" denotes that the modulation, i.e. inhibition or activation, of a channel not having the activity of a Kv1.7 containing channel is at least 10 or at least 20, more preferably at least 50, even more preferably at least 100, even more preferably at least 500 or at least 1000 and most preferably at least 10000 times lower than that of a channel comprising Kv1.7, preferably a channel consisting of Kv1.7.

"Metabolic diseases or conditions" is a summarizing term for diseases or conditions related to a malfunction of the metabolism of the body. Diseases falling under the term will be described in detail below and comprise metabolic syndrome, diabetes type 2, hypertension, central obesity, decreased HDL cholesterol and elevated triglycerides.

The term "secondary disease or condition" denotes a disease or condition that follows and results from an earlier disease. Secondary diseases or conditions related to metabolic diseases discussed further below are e.g. hypertension, diabetic retinopathy, neuropathy, cardiac infarction, peripheral vascular disease, apoplexia, nephropathy, stroke, diabetic foot, venous ulcer, amputation or blindness.

As evident upon comparison of both metabolic diseases or conditions and secondary diseases or conditions, some diseases or conditions are listed in both groups. In fact, the occurrence of one of the diseases or conditions listed as a secondary disease at a certain time point can be succeeded by that of another "secondary disease or condition" or by one falling under "metabolic disease or condition". All diseases listed in this invention, as apparent from the definitions below, are interrelated and the sequence of their occurrence may differ. Therefore, it depends on the individual case whether a specific disease is characterized as metabolic disease or condition or secondary disease or condition.

In the context of the present invention, it was found that Conkunitzin-S1 as represented by SEQ ID NO: 1 specifically modulates the activity of heterologuously expressed Kv1.7 potassium channels.

In contrast to the small fraction of potassium current arising from channels comprising Kv1.7 in β-cells (10 to 15%), the effect observed upon administration of Conkunitzin-S1 to said cells was remarkable in that it reduced the endogenous Kᵥ currents in these cells thus significantly enhancing insulin secretion, but without evident effects on ATP sensitive potassium channels (K_{ATP}). The observed increase in insulin secretion is small, especially at lower concentrations when measured in whole pancreatic islets, but significant for the entire range of glucose concentration tested at concentration of Conkunitzin-S1 of 10 µM in the cell medium. On the cellular level, Conkunitzin-S1 modulates the electrical excitability of pancreatic cells and leads to changes in the intracellular Ca²⁺-concentration.

Thus, without wishing to be bound by any scientific theory, the present inventors believe that Conkunitzin-S1, as well as (poly)peptides or peptidomometics derived therefrom which have the biological activity of Conkunitzin-S1, likely modulate glucose-mediated insulin secretion in pancreatic islets by inhibiting Kᵥ currents mediated by Kᵥ1.7. This modulatory effect of Conkunitzin-S1 on insulin secretion has also been shown *in vivo* in a rat whole animal glucose tolerance test (OGTT). In contrast to glibenclamide, a K_{ATP} antagonist interacting with sulfonylurea receptor subunits of ATP sensitive potassium channels, which is used for treatment of type 2 diabetes, no decrease in basal glucose levels is observed in the presence of Conkunitzin-S1. Accordingly, the effect of Conkunitzin-S1 on glucose stimulated insulin release is independent of K_{ATP} activity and does not result in hypoglycemia.

This observation was insofar unexpected that Conkunitzin-S1 was previously only known to inhibit channels of the *Drosophila Shaker family* and no mammalian target of Conkunitzin-S1 has been identified so far. Furthermore these data are the first report on a functional expression of human Kv1.7 channels.

Kv1.7 appears to be a minor component of the potassium currents present in pancreatic β-cells. Accordingly, the unexpected results obtained in the present invention that Conkunitzin-S1 specifically modulates Kv1.7 containing channels would not have directed the skilled person to investigate the possibility that a specific modulation on channels with an apparently only small role in directing potassium currents, i.e. those containing Kv1.7, could result in significant functional changes in insulin release. In addition, side effects comparable to those of sulfonylurea compounds, which reduce the glucose level to result in hypoglycemia, were surprisingly not observed.

Since Conkunitzin-S1 affects glucose induced insulin secretion without affecting basal glucose levels, the present results identify Kv1.7 containing channels as a suitable target for the treatment of metabolic diseases such as type 2 diabetes. This strategy offers a preferable alternative to the currently used therapies without immediate hypoglycemic risks.

In the context of the present invention, the (poly)peptide or peptidomimetic thereof according to the invention are to be administered in the form of a pharmaceutical composition.

In accordance with the present invention, the term "pharmaceutical composition" relates to a composition for administration to a patient, preferably a human patient. The pharmaceutical composition of the invention comprises a (poly)peptide or peptidomimetic according to the invention having the biological activity of Conkunitzin-S1, alone or in combination. It may, optionally, comprise further molecules capable of altering the characteristics of said (poly)peptide or peptidomimetic as defined above having the biological activity of Conkunitzin-S1 thereby, for example, stabilizing or modulating their function. The composition may be in solid, liquid or gaseous form and may be, *inter alia,* in the form of (a) powder(s), (a) tablet(s), (a) solution(s) or (an) aerosol(s). The pharmaceutical composition of the present invention may, optionally and additionally, comprise a pharmaceutically acceptable carrier. By "pharmaceutically acceptable carrier" is meant a non-toxic solid, semisolid or liquid filler, diluent, encapsulating material or formulation auxiliary of any type. Examples of suitable pharmaceutical carriers are well known in the art and include phosphate buffered saline solutions, water, emulsions, such as oil/water emulsions, various types of wetting agents, sterile solutions, organic solvents including DMSO etc. Compositions comprising such carriers can be formulated by well known conventional methods. The pharmaceutical composition can be administered in various ways, e.g. parenterally, nasally, sublingually, orally, percutaneously. The term "parenteral" as used herein refers to modes of administration, which include intravenous, which is particularly preferred, intramuscular, intraperitoneal, intrasternal, subcutaneous and intraarticular injection and infusion. The pharmaceutical composition can be administered to the subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the ordinary clinician or physician. Generally, the regimen as a regular administration of the pharmaceutical composition should be in the range of 1 µg to 5 g units per day. However, a more preferred dosage might be in the range of 0.01 mg to 100 mg, even more preferably 1 mg to 100 mg and most preferably 1 mg to 50 mg per day.

In a preferred embodiment, said (poly)peptide or peptidomimetic specifically inhibits the activity of a channel having the activity of a Kv1.7 containing channel.

As demonstrated in the appended examples, inhibition of the activity of channels comprising Kv1.7 was shown to occur under physiological conditions.

In a preferred embodiment, said metabolic disease is type 2 diabetes, metabolic syndrome, high blood pressure (hypertension), central obesity, decreased HDL cholesterol or elevated triglycerides.

Diabetes type 2 or type 2 Diabetes (formerly called non-insulin-dependent diabetes mellitus (NIDDM)) is a metabolic disorder that is primarily characterized by insulin resistance, relative insulin deficiency and hyperglycemia. It is often managed by increasing physical activity and dietary modification, although medications and insulin are often needed, especially as the disease progresses. The disease is now increasingly seen in children and adolescents, an increase thought to be linked to rising rates of obesity in this age group, although it remains a minority of cases.

Complex and multifactorial metabolic changes very often lead to damage and function impairment of many organs, most importantly the cardiovascular system in type 2 diabetes. This leads to substantially increased morbidity and mortality.

Unlike insulin-dependent diabetes mellitus (type 1), the insulin resistance in type 2 diabetes is generally "post-receptor", referring to a problem with the cells that respond to insulin rather than a problem with insulin production.

Important contributing factors are increased hepatic glucose production (e.g., from glycogen degradation), especially at inappropriate times (typical cause is aberrant insulin levels, as insulin controls this function in liver cells), decreased insulin-mediated glucose transport in (primarily) muscle and adipose tissues (receptor and post-receptor defects), impaired beta-cell function-loss of early phase of insulin release in response to hyperglycemic stimuli.

However, severe complications can result from improperly managed type 2 diabetes, including renal failure, blindness, slow healing wounds (including surgical incisions), and arterial disease, including coronary artery disease.

Apart from obesity, diabetes type 2 is often associated with hypertension, elevated cholesterol (combined hyperlipidemia), and with a condition often termed Metabolic syndrome (it is also known as Syndrome X, Reavan's syndrome, or CHAOS). Diabetes type 2 is also associated with acromegaly, Cushing's syndrome and a number of other endocrinological disorders. Additional factors found to increase risk of type 2 diabetes include aging, high-fat diets and a less active lifestyle (Eberhart et al., 2004).

Metabolic syndrome is a combination of medical disorders that increase the risk of developing cardiovascular disease and diabetes. It affects a great number of people, and prevalence increases with age. Metabolic syndrome is also known as metabolic syndrome X, syndrome X, insulin resistance syndrome or Reaven's syndrome. Symptoms and features are: Fasting hyperglycemia (diabetes type 2 or impaired fasting glucose, impaired glucose tolerance, or insulin resistance); hypertension; central obesity (also known as visceral, male-pattern or apple-shaped adiposity; overweight with fat deposits mainly around the waist); decreased HDL cholesterol or elevated triglycerides. Associated diseases and signs are: elevated uric acid levels, fatty liver (especially in concurrent obesity), progressing to non-alcoholic fatty liver disease, polycystic ovarian syndrome, hemochromatosis (iron overload); and acanthosis nigricans (a skin condition featuring dark patches). Different organizations dealing with diabetes have elaborated different definitions for metabolic syndrome. The World Health Organization (WHO) criteria (1999) require presence of diabetes type 2, impaired glucose tolerance, impaired fasting glucose or insulin resistance, and two of the following: blood pressure: ≥ 140/90 mmHg, dyslipidaemia: triglycerides (TG): ≥ 1.695 mmol/L and high-density lipoprotein cholesterol (HDL-C) ≤ 0.9 mmol/L (male), ≤ 1.0 mmol/L (female), central obesity: waist:hip ratio > 0.90 (male); > 0.85 (female), and/or body mass index > 30 kg/m², microalbuminuria: urinary albumin excretion ratio ≥ 20 mg/min or albumin:creatinine ratio ≥ 30 mg/g.

The US National Cholesterol Education Program (NCEP) Adult Treatment Panel III (2001) requires at least three of the following: central obesity (waist circumference ≥ 102 cm or 40 inches (male), ≥ 88 cm or 36 inches(female)), dyslipidaemia (TG ≥ 1.695 mmol/L (150 mg/dl), dyslipidaemia: HDL-C < 40 mg/dL (male), < 50 mg/dL (female)), high blood pressure (≥ 130/85 mmHg), fasting plasma glucose (≥ 6.1 mmol/L (110 mg/dl)).

The American Heart Association requires elevated waist circumference (Men - equal to or greater than 40 inches (102 cm); Women - equal to or greater than 35 inches (88 cm)); elevated triglycerides (equal to or greater than 150 mg/dL); Reduced HDL cholesterol (men - less than 40 mg/dL; women - less than 50 mg/dL); elevated blood pressure (equal to or greater than 130/85 mm Hg or use of medication for hypertension); elevated fasting glucose (equal to or greater than 100 mg/dL (5.6 mmol/L)) or use of medication for hyperglycemia.

Hypertension denotes an abnormally high blood pressure. Based on recommendations of the Seventh Report of the Joint National Committee of Prevention, Detection, Evaluation, and Treatment of High Blood Pressure (JNC VII), the classification of blood pressure (expressed in mm Hg) for adults aged 18 years or older is as follows*: Normal - Systolic lower than 120, diastolic lower than 80; Prehypertension - Systolic 120-139, diastolic 80-99; Stage 1 - Systolic 140-159, diastolic 90-99; Stage 2 - Systolic equal to or more than 160, diastolic equal to or more than 100. Hypertension may be either essential or secondary. Essential hypertension is diagnosed in the absence of an identifiable secondary cause. Approximately 95% of American adults have essential hypertension, while secondary hypertension accounts for fewer than 5% of the cases.

An elevation of the systolic and/or diastolic blood pressure increases the risk of developing heart (cardiac) disease, kidney (renal) disease, hardening of the arteries (atherosclerosis or arteriosclerosis), eye damage, and stroke (brain damage). These complications of hypertension are often referred to as end-organ damage because damage to these organs is the end result of chronic high blood pressure.

Central obesity is associated with a statistically higher risk of heart disease, hypertension, insulin resistance, and diabetes mellitus type 2. Belly fat is a symptom of metabolic syndrome, and is an indicator used in the diagnosis of that disorder.

There are numerous theories as to the exact cause and mechanism in type 2 diabetes. Central obesity is known to predispose individuals for insulin resistance. Abdominal fat is especially active hormonally, secreting a group of hormones called adipokines that may possibly impair glucose tolerance.

Insulin resistance is a major feature of diabetes mellitus type 2, and central obesity is correlated with both insulin resistance and T2DM itself. Increased adiposity (obesity) raises serum resistin levels, which in turn directly correlate to insulin resistance. Studies have also confirmed a direct correlation between resistin levels and T2DM. And it is waistline adipose tissue (central obesity) which seems to be the foremost type of fat deposits contributing to rising levels of serum resistin. Conversely, serum resistin levels have been found to decline with decreased adiposity following medical treatment.

In another preferred embodiment, said secondary disease or condition related to said metabolic diseases is high blood pressure, diabetic retinopathy, neuropathy, cardiac infarction, peripheral vascular disease, nephropathy, stroke, diabetic foot, venous ulcer, amputation or blindness.

Neuropathy is used as a shortened term for peripheral neuropathy. Peripheral neuropathy is defined as aberrant function and structure of peripheral motor, sensory, and autonomic neurons, involving either the entire neuron or selected levels. The four cardinal patterns of peripheral neuropathy are polyneuropathy, mononeuropathy, mononeuritis multiplex and autonomic neuropathy. The most common form is (symmetrical) peripheral polyneuropathy, which mainly affects the feet and legs.

The common causes of painful peripheral neuropathies are diabetes and other metabolic conditions (Portenoy, 1989; Vaillancourt and Langevin, 1999).

Diabetic retinopathy is retinopathy (damage to the retina) caused by complications of diabetes mellitus, which can eventually lead to blindness. It is an ocular manifestation of systemic disease which affects up to 80% of all patients who have had diabetes for 10 years or more.

Cardiac infarction (AMI or MI), occurs when the blood supply to parts of the heart is interrupted. This is most commonly due to occlusion (blockage) of a coronary artery following the rupture of a vulnerable atherosclerotic plaque, which is an unstable collection of lipids (like cholesterol) and white blood cells (especially macrophages) in the wall of an artery. The resulting ischemia (restriction in blood supply) and oxygen shortage, if left untreated for a sufficient period, can cause damage and/or death (infarction) of heart muscle tissue (myocardium). Important risk factors are previous cardiovascular disease (such as angina, a previous heart attack or stroke), older age (especially men over 40 and women over 50), tobacco smoking, high blood levels of certain lipids (triglycerides, low-density lipoprotein or "bad cholesterol") and low high density lipoprotein (HDL, "good cholesterol"), diabetes, high blood pressure, obesity, chronic kidney disease, excessive alcohol consumption, the abuse of certain drugs (such as cocaine), and chronic high stress levels (Bax et al., 2008; Pearte et al., 2006).

Peripheral vascular disease (PVD), also known as peripheral artery disease (PAD) or peripheral artery occlusive disease (PAOD), is a collator for all diseases caused by the obstruction of large peripheral arteries, which can result from atherosclerosis, inflammatory processes leading to stenosis, an embolism or thrombus formation. It causes either acute or chronic ischemia (lack of blood supply), typically of the legs.

One major cause is Diabetes type 2 which causes endothelial and smooth muscle cell dysfunction in peripheral arteries. Up to 70% of nontraumatic amputations are performed on diabetic patients, and a known diabetic who smokes runs an approximately 30% risk of amputation within 5 years. Another cause is dyslipidemia, i.e. the elevation of total cholesterol, LDL cholesterol, and triglyceride levels. Correction of dyslipidemia by diet and/or medication is associated with a major improvement in short-term rates of heart attack and stroke. This benefit is gained even though current evidence does not demonstrate a major reversal of peripheral and/or coronary atherosclerosis.

Hypertension, i.e. elevated blood pressure, is correlated with an increase in the risk of developing peripheral artery disease, as well as with associated coronary and cerebrovascular events (heart attack and stroke). Risk of peripheral artery occlusive disease increases if the patient in addition to suffering from hypertension is obese, or has a personal history of vascular disease, heart attack, or stroke.

Microvascular disease or microangiopathy is a disease process affecting small blood vessels in the body. The disease sometimes occurs when a person has had diabetes type 2 for a long time. High blood glucose levels cause the endothelial cells lining the blood vessels to take in more glucose than normal (these cells do not depend on insulin). They then form more glycoproteins on their surface than normal, and also cause the basement membrane to grow thicker and weaker. The walls of the vessels become abnormally thick but weak, and therefore they bleed, leak protein, and slow the flow of blood through the body. Then some cells, for example in the retina (diabetic retinopathy) or kidney (diabetic nephropathy), may not get enough blood and may be damaged. Nerves, if not sufficiently supplied with blood, are also damaged which may lead to loss of function (diabetic neuropathy).

Nephropathy is the medical term for diseases of the kidney or kidney function. Diabetic nephropathy is a secondary disease of diabetes type 2. The disease pattern is not clearly defined, but is the sum of different alterations caused by the diabetic metabolism in the kidneys, among them inflammation, alterations in the blood vessels, disease of the filter apparatus of the kidney. In some cases kidney diseases lead to hypertension (hypertensive nephropathy) which in turn can lead to further damage to the kidney.

Stroke is the rapidly developing loss of brain functions due to a disturbance in the blood vessels supplying blood to the brain. This can be due to ischemia (lack of blood supply) caused by thrombosis or embolism, or due to a hemorrhage. Risk factors for stroke include advanced age, hypertension, previous stroke or transient ischemic attack (TIA), diabetes, high cholesterol, cigarette smoking, atrial fibrillation, the contraceptive pill, migraine with aura, and thrombophilia (a tendency to thrombosis). Hypertension is the most important modifiable risk factor of stroke.

Diabetic foot is an umbrella term for foot problems in patients with diabetes mellitus. Due to arterial abnormalities and diabetic neuropathy, as well as a tendency to delayed wound healing, infection or gangrene of the foot is relatively common. 10 to 15% of diabetic patients develop foot ulcers at some point in their lives and foot related problems are responsible for up to 50% of diabetes related hospital admissions.

Venous ulcers (or varicose ulcers) are wounds that are thought to occur due to improper functioning of valves in the veins, usually of the legs. They are the major cause of chronic wounds, occurring in 70% to 90% of chronic wound cases. The exact etiology of venous ulcers is not certain, but they are thought to arise when venous valves, that exist to prevent backflow of blood, do not function properly, causing the pressure in veins to increase. The body needs the pressure gradient between arteries and veins in order for the heart to pump blood forward through arteries and into veins. When venous hypertension exists, arteries no longer have significantly higher pressure than veins, blood is not pumped as effectively into or out of the area, and arterial and venous blood is mixed.

In a different embodiment, the present invention relates to a method of screening for (poly)peptides derived from Conkunitzin-S1 suitable for specifically modulating the activity of a channel having the activity of a Kv1.7 containing channel, comprising: (a) altering the amino acid sequence of Conkunitzin-S1 represented by SEQ ID NO: 1 by deleting and/or inserting and/or replacing at least one amino acid; and (b) determining the modulatory effect of the (poly)peptide, obtained in step (a) (i) on a channel having the activity of a Kv1.7 containing channel and (ii) on channels, preferably potassium channels, not having the activity of a Kv1.7 containing channel which are optionally expressed on the same cell as the channel having the activity of a Kv1.7 containing channel; wherein a modulatory effect of the (poly)peptide determined in step (i) that is at least 50% of the modulatory effect of Conkunitzin-S1 indicates that the (poly)peptide is suitable for modulating the activity of said channel having the activity of a Kv1.7 containing channel; and wherein the determination of essentially no modulatory effect in step (ii) indicates that the (poly)peptide is specifically modulating the activity of channels having the activity of a Kv1.7 containing channel.

Altering the amino acid sequence of Conkunitzin-S1 represented by SEQ ID NO: 1 by deleting and/or inserting and/or replacing/substituting at least one amino acid refers to the mutation of the amino acid sequence of Conkunitzin-S1. Alterations are not restricted to one site in the polypeptide, but simultaneous insertion, deletion or substitution of at least one amino acid at more than one site in any combination possible are envisaged as well. For example, an insertion of one or more amino acids may be effected at one site and a substitution of one or more amino acids may be effected at a different site in the sequence of the polypeptide. Equally applicable combinations are insertions at multiple sites, deletions at multiple sites or substitutions at multiple sites, each effected with one or more amino acids. Specific examples of such combinations are duplications of one or more amino acids or inversions, i.e. the deletion of two or more amino acids and the insertion of said amino acids in inverted sequence.

It is preferred that the sequence of the resulting (poly)peptide has at least 85% sequence identity to SEQ ID NO: 1.

The determination of the modulatory effect of the (poly)peptide obtained in step (a) can be effected by various methods known to the skilled person. Suitable methods are disclosed in the appended examples and comprise electrophysiological measurements on heterologously expressed channels (e.g. in Xenopus oocytes) and electrophysiological measurements on isolated cells (Stühmer (1992); Stühmer et al. (1992)).

In this regard, a modulatory effect regarded as significant is at least 50% of the modulatory effect of Conkunitzin-S1, preferably at least 75%, more preferably at least 85, at least 95 or at least 98% and even more preferably the modulatory effect is at least as strong as the modulatory effect of Conkunitzin-S1. It is most preferred that the modulatory effect exceeds the modulatory effect of Conkunitzin-S1, i.e. the modulatory effect may be at least 120%, at least 150% or at least 200%.

Determining the modulatory effect of the (poly)peptide obtained in step (a) on potassium channels not having the activity of a Kv1.7 containing channel, which are optionally expressed on the same cell as Kv1.7, serves to secure that the (poly)peptide modulates channels having the activity of a Kv1.7 containing channel and does essentially not have any modulatory effect on other channels not having the activity of a Kv1.7 containing channel which could result either in unwanted side effects or in an attenuation of the desired effect. In this regard, the term "essentially no modulatory effect" in connection with potassium channels not having the activity of a Kv1.7 containing channel defines an effect that is at least 10 or at least 20 times lower than that observed for channels having the activity of a Kv1.7 containing channel, preferably at least 50 times lower, more preferably at least 100 times lower, even more preferably at least 500 times lower and most preferably at least 1000 times lower than that observed for channels having the activity of a Kv1.7 containing channel.

In a preferred embodiment, the potassium channels not having the activity of a Kv1.7 containing channel are expressed in pancreatic β-cells, cardiac cells, skeletal muscle cells, kidney cells, smooth muscle cells or liver cells. These are the cell types which also express channels having the activity of a Kv1.7 containing channel. Therefore, a comparison of the effect of a candidate (poly)peptide on channels having the activity of a Kv1.7 containing channel in a specific cell with other channels not having the activity of a Kv1.7 containing channel in the same cell is of particular use in the present invention. Pancreatic β-cells are particularly preferred in connection with a possible treatment of metabolic diseases, in particular type 2 diabetes, with a product identified with the method of the present invention. Type 2 diabetes is characterized by a reduced or abolished insulin secretion from pancreatic β-cells. Therefore, to exclude or reduce side effects of the (poly)peptide identified on the secretion of insulin, one or more, preferably all (potassium) channels present on pancreatic β-cells responsible for insulin secretion can be examined. It is equally preferred that the channels having the activity of a Kv1.7 containing channel are expressed on pancreatic β-cells.

Based on sequence homology of the hydrophobic transmembrane cores, the alpha subunits of voltage-gated potassium channels have been grouped into 12 classes labeled Kᵥ1-12. Examples of Kv channels include delayed rectifier channels (Kᵥα1.x - Shaker-related: Kᵥ1.1 (KCNA1), Kᵥ1.2 (KCNA2), Kᵥ1.3 (KCNA3), Kᵥ1.4 (KCNA4), Kᵥ1.5 (KCNA5), Kᵥ1.6 (KCNA6), Kᵥ1.7 (KCNA7), Kᵥ1.8 (KCNA8); Kᵥα2.x - Shab-related: Kᵥ2.1; (KCNB1), Kᵥ2.2 (KCNB2); Kᵥα3.x - Shaw-related: Kᵥ3.1 (KCNC1), Kᵥ3.2 (KCNC2); Kᵥα7.x: Kᵥ7.1 (KCNQ1) - KvLQT1, Kᵥ7.2 (KCNQ2), Kᵥ7.3 (KCNQ3), Kᵥ7.4 (KCNQ4), Kᵥ7.5 (KCNQ5) and Kᵥα10.x: Kᵥ10.1 (KCNH1)), A-type potassium channels (Kᵥα3.x - Shaw-related: Kᵥ3.3 (KCNC3), Kᵥ3.4 (KCNC4); Kᵥα4.x - Shal-related: Kᵥ4.1 (KCND1), Kᵥ4.2 (KCND2), Kᵥ4.3 (KCND3)); outward rectifying channels (Kᵥα10.x: Kᵥ10.2 (KCNH2)); inward rectifying channels (Kᵥα11.x - ether-ago-go potassium channels: Kᵥ11.1 (KCNH2) - hERG, Kᵥ11.2 (KCNH6), Kᵥ11.3 (KCNH7)); slowly activating channels (Kᵥα12.x: Kᵥ12.1 (KCNH8), Kᵥ12.2 (KCNH3), Kᵥ12.3 (KCNH4)) and modifier/silencer channels which are unable to form functional channels as homotetramers but instead heterotetramerize with Kᵥα2 family members to form conductive channels (Kᵥα5.x: Kᵥ5.1 (KCNF1); Kᵥα6.x: Kᵥ6.1 (KCNG1), Kᵥ6.2 (KCNG2), Kᵥ6.3 (KCNG3), Kᵥ6.4 (KCNG4); Kᵥα8.x: Kᵥ8.1 (KCNV1), Kᵥ8.2 (KCNV2) and Kᵥα9.x: Kᵥ9.1 (KCNS1), Kᵥ9.2 (KCNS2), Kᵥ9.3 (KCNS3)). Do date it is not known in detail which subunits may combine to form functional conducting pores, in particular not which subunits may combine with Kv1.7 subunits.

Examples of potassium channels which can be comparatively investigated in the context of the present invention for their sensitivity to the (poly)peptides obtainable with the method of the present invention are those comprising Kv2.1 or Kv2.2. Other potassium channels not having the activity of a Kv1.7 containing channel consist of the other members of the mammalian homologues of the *Shaker* channel, i.e. Kv1.1 to Kv1.6. These channels can be investigated for their sensitivity to said (poly)peptides if they do not comprise Kv1.7 or a derivative thereof being sensitive to Conkunitzin-S1 as defined above.

The investigation of the sensitivity to the (poly)peptides with the method of the present invention is not restricted to potassium channels but can also be extended to other ion channels such as Na⁺- or Ca⁺⁺-channels and other membrane proteins with similar structures, such as Trp channels.

In a preferred embodiment, the potassium channels other than those having the activity of a Kv1.7 containing channel are channels consisting of Kv2.1 and/or 2.2.

In another preferred embodiment, the (poly)peptide identified in the method of the present invention inhibits the activity of channels having the activity of a Kv1.7 containing channel.

An inhibitory effect of Conkunitzin-S1 is demonstrated herein in the examples of heterologously expressed Kv1.7 forming channels and on Kv1.7 forming channels present in pancreatic β-cells.

In a further preferred embodiment, said channel having the activity of a Kv1.7 containing channel consists of Kv1.7 subunits.

In order to provide a suitable and reworkable control, a homotetrameric channel can be used in the present method. This is in particular the case if cells heterologously expressing Kv1.7 channels are used in the method of the present invention.

In a preferred embodiment, the method of the invention further comprises optimizing the pharmacological properties of a (poly)peptide identified as specifically modulating the activity of channels having the activity of a Kv1.7 containing channel.

Methods for the optimization of the pharmacological properties of compounds identified in screens, generally referred to as lead compounds, are known in the art and comprise a method of modifying a compound identified as a lead compound to achieve: (a) modified site of action, spectrum of activity, organ specificity, and/or (b) improved potency, and/or (c) decreased toxicity (improved therapeutic index), and/or (d) decreased side effects, and/or (e) modified onset of therapeutic action, duration of effect, and/or (f) modified pharmacokinetic parameters (absorption, distribution, metabolism and excretion), and/or (g) modified physico-chemical parameters (solubility, hygroscopicity, color, taste, odor, stability, state), and/or (h) improved general specificity, organ/tissue specificity, and/or (i) optimized application form and route by a. esterification of carboxyl groups, or b. esterification of hydroxyl groups with carboxylic acids, or c. esterification of hydroxyl groups to, e.g. phosphates, pyrophosphates or sulfates or hemi-succinates, or d. formation of pharmaceutically acceptable salts, or e. formation of pharmaceutically acceptable complexes, or f. synthesis of pharmacologically active polymers, or g. introduction of hydrophilic moieties, or h. introduction/exchange of substituents on aromates or side chains, change of substituent pattern, or i. modification by introduction of isosteric or bioisosteric moieties, or j. synthesis of homologous compounds, or k. introduction of branched side chains, or I. conversion of alkyl substituents to cyclic analogues, or m. derivatization of hydroxyl group to ketales, acetales, or n. N-acetylation to amides, phenylcarbamates, or o. synthesis of Mannich bases, imines, or p. transformation of ketones or aldehydes to Schiff's bases, oximes, acetales, ketales, enolesters, oxazolidines, thiazolidines; or cyclisation, or allowing post-translational modifications; or combinations thereof.

The various steps recited above are generally known in the art. They include or rely on quantitative structure-action relationship (QSAR) analyses (Kubinyi, 1992), combinatorial biochemistry, classical chemistry and others (see, for example, Holzgrabe and Bechtold, 2000).

Cyclic (poly)peptides are (poly)peptide chains whose amino and carboxyl termini are themselves linked together with a peptide bond, forming a circular chain. One interesting property of cyclic (poly)peptides is that they tend to be extremely resistant to digestion, allowing them to survive intact in the human digestive tract. This trait makes cyclic (poly)peptides attractive to protein based drug designers for use as scaffolds which, in theory, could be engineered to incorporate any arbitrary protein or peptide domain of medicinal value, in order to allow those components to be delivered orally. In context of the present invention, cyclization is a particularly preferred modification to a (poly)peptide identified with the method of the present invention.

As described above, Conkunitzin-S1 is naturally post-translationally modified, i.e. amidated. The present inventors could express Conkunitzin-S1 in E.coli (Bayerhuber et al., 2006) which means that the expressed Conkunitzin-S1 is not amidated. However, said lack of amidation was shown not to be essential for the biological activity of the polypeptide. Nevertheless, expression or synthesis of (poly)peptides according to the present invention or identified with the method of the present invention is preferably effected such that post-translational modifications are effected to potentially increase the biological activity of the recombinantly expressed or chemically synthesized (poly)peptide. This can also include modifications not naturally effected to Conkunitzin-S1, such as glycosylation or phosphorylation.

The figures show:

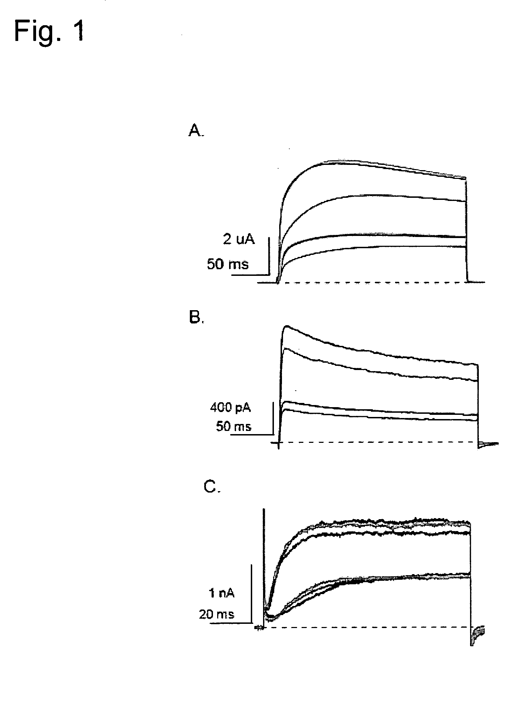

**Fig. 1** Conkunitzin-S1 blocks Kv1.7 and delayed rectifier currents from isolated rat pancreatic islet cells. Currents were elicited by 150-250 ms pulses to 0 and 40 mV (Vh= -80mV). Black is control, dark grey Conkunitzin-S1 (Conk-S1), and light grey is wash. **A.** Mouse Kv1.7 currents through channels expressed in Xenopus oocytes exposed to 250 nM Conk-S1. **B.** Human Kv1.7 currents through channels expressed HEK cells exposed to 250 nM Conk-S1. C. Delayed rectifier currents from rat pancreatic islet insulin/Kv1.7 positive cells exposed to 500 nM Conk-S1.

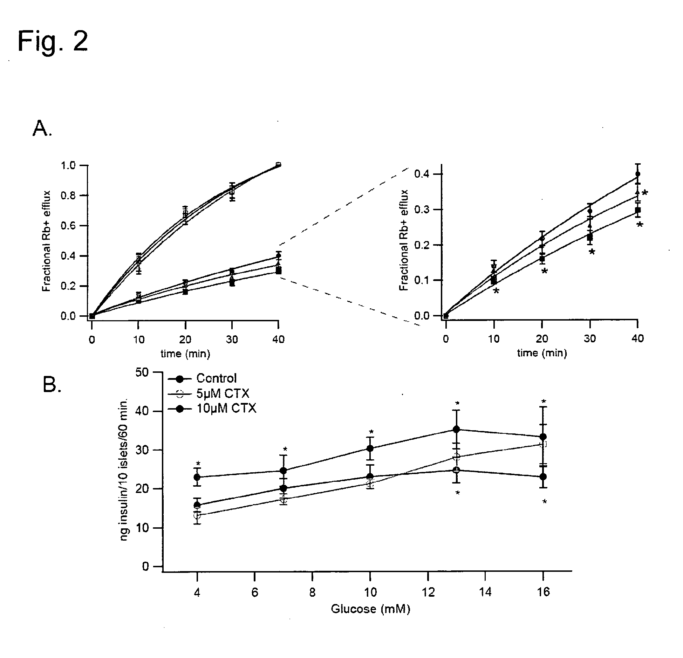

**Fig. 2** Conkunitzin-S1 modulates glucose stimulated insulin secretion from rat pancreatic islets through block of Rb+ effluxes through Kv but not KATP channels. A. Fractional 86Rb+ efflux in the presence and absence of Conk-S1 as a function of time from representative islet samples. For KATP MI contained: 2.5 mg/ml oligomycin, 1 mM 2-deoxyglucose, 10 mM TEA, 10 µM nifedipine and 30 mM KCI, black circles. For Kv channel assay, MI solution contained 10 mM D(+)glucose, 1 µM glibenclamide, and 30 mM KCI. (mean ± sem of 3-5 independent determinations from islets isolated from different animals). • is control, 1 µM Conk △, 10 µM Conk-S1 is ■. Inset show only the Rb fluxes through Kv channels. (mean ± sem; n= 3-8 independent determinations from islets isolated from different animals). B. Insulin secretion from rat pancreatic islets stimulated with increasing concentration of glucose in the presence of 5 uM (open circles) and 10 µM (closed circles) Conk-S1. (mean ± sem; n= 3 independent determinations in triplicate from islets isolated from different animals).

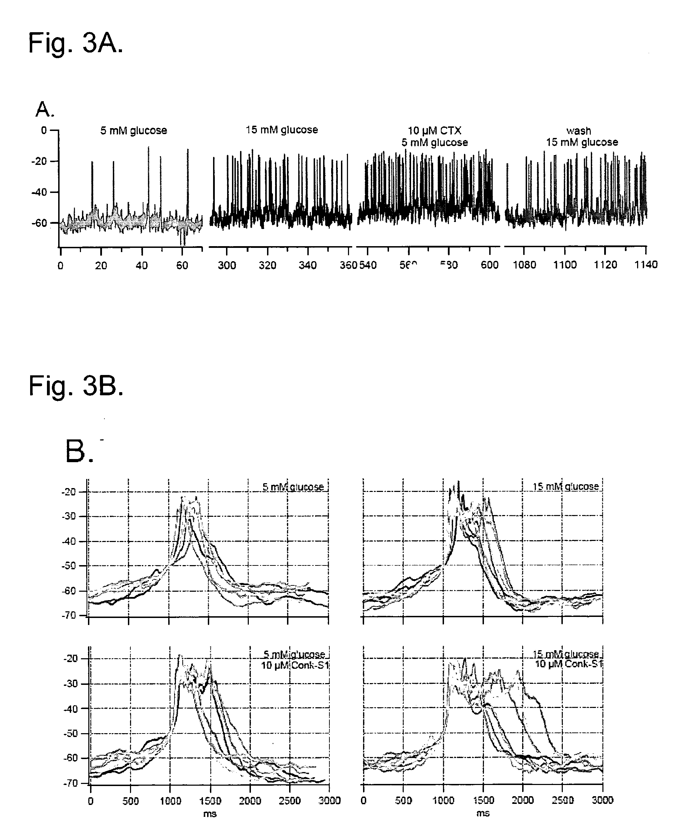

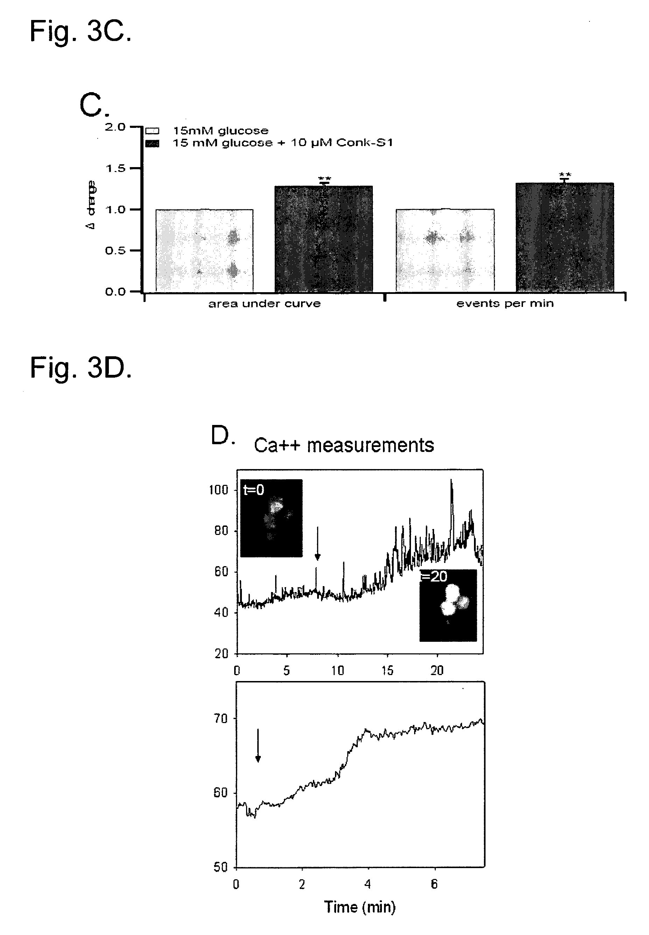

**Fig. 3** Glucose stimulated action potentials and intracellular calcium concentration increase from rat pancreatic islet cells are modulated by Conk-S1**. A.** Train of APs elicited by glucose stimulation with and without Conk-S1. Representative spike shape in the conditions shown above (except 5 mM glucose, 10 µM Conk-S1). **B.** Spike width comparison. Each panel shows 10 spikes in the presence of 5 and 15 mM glucose with and without Conk-S1. For comparison spikes were aligned at the point crossing -50mV. C. Quantification of Conk-S1 effect on rat islet cell APs (n=5). D. Intracellular Ca⁺² measurements from isolated rat islet cells loaded with fluo4. Upper panel shows representative calcium oscillations in response to 15 mM glucose and addition of 10 µM Conk-S1 (4 fast flickering cells). The insets show control Fluo4 signal at t=0 for control and after ∼10 min in Conk-S1. Lower panel spontaneous increase in the intracellular calcium concentration of no/or slow flickering cells in response to 10 µM Conk-S1 addition. The arrows show the addition of Conk-S1.

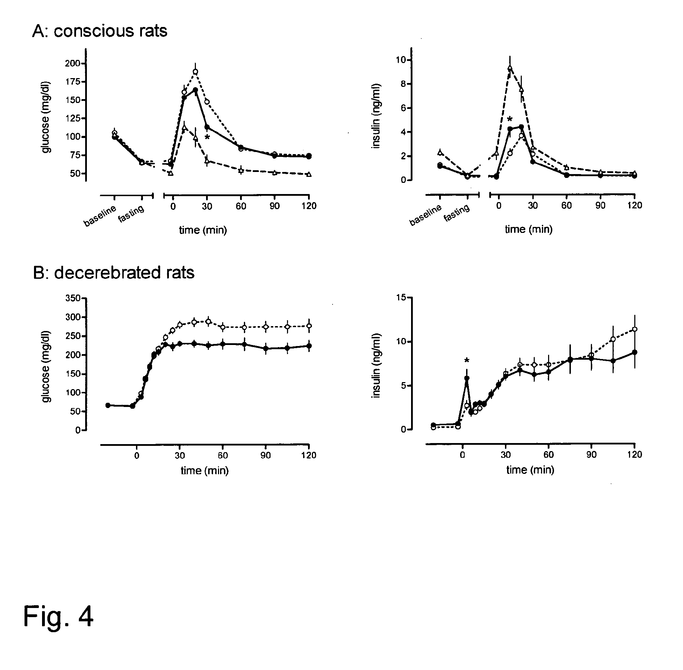

**Fig. 4** Conk-S1 modules insulin secretion and glucose levels in vivo . **A.** Glucose tolerance test of conscious animals. Influence of Conk-S1 (•, 100 nmol/kg i.v. 130 min before glucose challenge) and glibenclamide (△, 0,3 mg/kg i.v. 10 min before glucose challenge) compared to controls (○) n glucose and insulin levels in rats after an OGTT (1g/kg p.o.). Left panel blood glucose levels. Right Panel insulin levels.
The AUC that has been calculated from the glucose profiles were diminished by glibenclamide (79±7g/l•min; p<0.01) and Conk-S1 (118±3 g/l•min; p<0.05) when compared to the controls (132±3 g/l•min). The AUC of the concerning insulin profiles was simultaneously increased only by glibenclamide (133±15 vs. 310±35 ng/ml•min). However, plasma insulin levels were enhanced in Conk-S1 treated animals 10 min after the glucose challenge. means±SEM, n=6-14.

**B.** Glucose clamp on decerebrated rats. Right panel blood glucose levels and left panel shows insulin levels. Influence of Conk-S1 (•, 100 nmol/kg i.v. as a bolus 240 min before glucose clamp+100 nmol/kg as a maintenance dosage within 4h) on glucose and insulin levels after glucose clamps (8,988 mg/min) compared to controls (○).

Note that the glucose levels were decreased by Conk-S1 compared to controls (○) in response to the glucose clamp. Insulin sharply increased just after glucose infusion (2.75 ± 0.56 vs. 5.84 ± 1.02 ng/ml; p<0.05;) and stabilized afterwards on control levels. means±SEM, n= 7-10.

The examples illustrate the invention.

### Example 1: Materials and Methods

All cloning strategies including sequence analysis, primer design, restriction mapping, and sequence alignments were assisted by online analysis with the Laser gene software (DNAstar, USA).

### Cloning of KCNA 7 (hKv1.7)

One step RT-PCR (Advantage RT-PCR kit, Invitrogen), using the standard conditions suggested by the manufacturer, was performed using human heart total RNA (Clontech) as template. The primers were designed according to the sequence published by Kashuba et al. (2001) under the accession number AJ310479. Cycling conditions: cDNA synthesis: 45-60°C for 15-30 min; denaturation: 94°C for 2 min. PCR amplification 40 cycles: denature at 94°C for 15s; annealing at 55-60°C for 30s; extenssion at 68°C for 1 min. Final extension 68°C for 5 min.

The full length construct was subcloned in the *Xenopus laevis* oocyte expression vector pSGEM (Liman et al. 1992). cDNA constructs were linearized with Nhe I and transcribed invitro with the T7 Polymerase (Stratagene) rendering capped cRNAs.

Functional analysis of human Kv1.7 α-subunits was performed in the *Xenopus laevis* oocyte heterologous expression system. Oocytes were surgically removed from anesthetized female *Xenopus laevis* specimens (20-30 min in 1.25 g/l Tricaine solution). Defoliculation was performed by partial enzymatic digestion with Collagenase type 2 (440 µ/ml, Worthington Biochemical Corporation, USA). Washes and storage were done on Barth medium: 88mM NaCl, 1mM KCI, 7.5mM Tris-HCl, 2.4mM NaHCO3, 0.82mM MgSO4, 0.33mM Ca(NO₃)₂, 0.41 mM CaCl2, osmolarity 230-240mosmols, pH 7.4 adjusted with NaOH. Microinjection: oocytes from stages IV-VI were injected with ∼750pgr cRNA (in 50nl) and incubated at 17.4°C in antibiotic supplemented Barth medium 24-72hr prior to electrophysiological analysis.

The antibiotics used were Cefuroxim/Zinacef750 (4mg/l, Aventis, France) or Penicillin/Streptomycin (100 U/ml, Gibco, USA).

Two electrode voltage clamp recordings (TEVC) were performed with a Turbo TEC-10 amplifier (Turbo Tec, npi electronics, Tamm, Germany) with electronic built-in series resistance compensation. The electrical stimulation and registration of the current was performed through the EPC9 built-in ITC-16 AD/DA converter, controlled by a Macintosh G4 computer (Apple computer, Cupertino, Ca, USA). The acquisition of data was made using Pulse software (HEKA, Lambrecht/Pfalz, Germany). TEVC microelectrodes were made from borosilicate filament glass capillaries (Hilgenberg, Germany), coated with RTV (GE Bayer Silicones, The Netherlands), and filled with 2M KCI. TEVC currents were subtracted online with a standard P/n protocol. TEVC current signals were sampled at 250-100 µs (sampling rate: 4-10kHz), low pass filtered with a Bessel filter at a frequency 4 times lower than the inverse of the sampling time, 1-2.5kHz. The standard extracellular bath solution was normal frog Ringer (NFR) containing 115mM NaCl, 2.5 mM KCI, 1.8mM CaCl2, 10mM HEPES-NaOH (pH7.2). The holding potential (Vh) was -100 mV in all experiments. The pulse interval between two stimuli was always kept long enough to ensure re-equilibration of the channel states and recovery from inactivation. All experiments were performed at room temperature (20-22°C).

Data acquisition was performed with the Pulse/PulseFit software package (HEKA Elektronik, Lambrecht, Germany). Off-line analysis was performed with a Macintosh G4 microcomputer (Apple, Cupertino, CA, U.S.A.) with Pulse Fit (HEKA, Germany) and Igor (Wavemetrics, Lake Oswego, USA) softwares.

Recombinant Conkunitzin-S1 was produced by using a bacterial expression system as described in Bayrhuber et al. (2006).

All data is expressed as mean ± standard error. Two tailed t-tests were used to evaluate the significance of the difference between means (P < 0.05) (Gossett, 1958).

***Islet isolation.*** Adult male Sprague-Dawley rats (Harlan Sprague-Dawley, Indianapolis, IN; ∼200) were anesthetized with isoflurane (0.6 ml) in an anesthetizing chamber and killed by cervical dislocation. Pancreata were removed and injected with Hank's balanced salt solution (Sigma Corp., St. Louis) containing collagenase (0.3 mg/ml) (pH 7.4) Collagenase Type XI was obtained from Sigma Corp. (St. Louis, Mo.). Pancreata were digested for 5 min at 37°C, hand shaken and washed 3 times in cold Hank's solution (Remedi et al, 2004). Islets were isolated by hand under a dissecting microscope and pooled islets were maintained in CMRL-1066 culture medium (GIBCO) supplemented with fetal calf serum (FCS, 10 %), penicillin (100 units/ml), and streptomycin (100 µg/ml).

***⁸⁶Rb⁺ Efflux Experiments.*** Isolated islets were pre-incubated with ⁸⁶Rb⁺ (rubidium chloride 1.5 mCi/ml, Amersham Biosciences) for 1 hr. Loaded islets were placed in microcentrifuge tubes (30 per group) and washed twice with RPMI-1640 media (Sigma Corporation). ⁸⁶Rb⁺ efflux was assayed by replacing the bathing solution with Ringer's solution with metabolic inhibitor (MI) with or without 0.5, 1 and 10 µmol/l conkunitzin. For K_{ATP} channel assay, MI solution contained 2.5 mg/ml oligomycin, 1 mM. 2-deoxyglucose, together with 10 mM tetraethylammonium to block voltage-gated K⁺ channels, 10 µM nifedipine to block Ca²⁺ entry and 30 mM KCI to maintain Em ∼ 0 (Remedi et al, 2006). For Kv channel assay, MI solution contained 10 mM D(+)glucose, 1 µM glibenclamide to block K⁺ channel and 30 mM KCI to maintain Em ∼ 0. The bathing solution was replaced with fresh solution every 10 min over a 40 min. period, and counted in a scintillation counter. ⁸⁶Rb-efflux was fit by a single exponential and the reciprocal of the exponential time constant (rate constant) for each efflux experiment is then proportional to the K⁺ (Rb⁺)-conductance of the islet membranes (Remedi et al, 2004 and 2006).

*Insulin Release Experiments.* Following overnight incubation in low glucose (5.6 mM) CMRL-1066 Medium, islets (10 per well in 12 well plates) were pre-incubated for 30 min in glucose-free CMRL-1066 plus 3 mM D(+)glucose, then incubated in CMRL-1066 plus different glucose concentration with or without conkunitzin, as indicated. Islets were incubated for 60 min at 37°C and medium removed and assayed for insulin content. Insulin was measured using Rat Insulin radioimmunoassay according to manufacturer's procedure (Linco Inc., St. Charles, MO). The results are from 3 independent experiments repeated in triplicate.

OGTT: Chronic polyethylene catheters were inserted during pentobarbitone anaesthesia (75 mg/kg) into the right femoral vein and artery for drug administration and collecting blood samples. Catheters were tunneled under the back skin, exteriorized in the region of the cervical vertebra and fixed at the skin. OGTTs were performed 3 days after catheterization in rats after fasting for 16h.

Decerebrated animals: The medulla and thoracolumbar portion of the spinal cord of the rats were destroyed using a steel pithing rod (1.5 mm diameter). Both vagal nerves were cut at the neck, and neuromuscular junctions were blocked using d-tubocurarine (3 mg/kg). Polyethylene catheters were inserted into both femoral veins (PE-10) for drug administration and into both carotid arteries (PE-50) for measuring blood pressure and collecting blood samples.

### Example 2:

To date, no specific blocker of Kᵥ1.7-mediated currents has been available. Recently, we reported that the conopeptide Conkunitzin-S1 (Conk-S1) from the venom of the predatory cone snail *Conus striatus* blocks voltage activated *Shaker* channels from Drosophila. Here, we report the action of this conopeptide on heterologously expressed mammalian channels. Fig. 1 a shows that Conk-S1 blocked mouse Kᵥ1.7 channels in *Xenopus* oocytes with an IC₅₀ of 122±11 nM (n=4). Human Kᵥ1.7 channels were blocked by Conk-S1 with an IC₅₀ of 445±50 nM when expressed in HEK-293 cells (n=11) (Fig. 1b). In contrast to Kᵥ1.7 channels, other voltage activated potassium channels (Kᵥ1.1 - Kᵥ1.6; Kᵥ3.1; Kᵥ3.2; Kᵥ3.4; reag1; reag2) expressed in *Xenopus* oocytes were not affected by 1 µM Conk-S1 (data not shown). 10 µM of Conk-S1. did not affect currents from oocytes injected with Kᵥ2.1, or Kv2.2 cRNAs. These results establish Conk-S1 as a specific antagonist of Kᵥ1.7 channels.

In situ hybridization has demonstrated the presence of Kᵥ1.7 in mouse pancreatic islet cells (Kalman et al., 1998). To investigate the potential effects of Conk-S1 on the electrical activity of isolated pancreatic β-cells the evoked Kᵥ currents of these cells were measured in the presence and absence of Conk-S1 using whole cell patch clamp. Fig. 1c shows that Conk-S1 reduced the endogenous Kᵥ currents in these cells. Conk-S1 (0.5 uM) blocked 18±0.02 % (n=8 ) of the total delayed rectifier currents at 0 and 40 mV recorded from rat islet cells that were positive to single cell PCR for insulin and Kv1.7 transcripts (data not shown). This indicates that only a minor component of the voltage activated K⁺ currents present in these cells is sensitive to this conopeptide.

To determine whether this small but consistent decrease in Kᵥ currents might have functional importance, whole rat islets were prepared and the voltage-dependent and K_{ATP}-dependent currents were measured by means of Rb⁺-fluxes in vitro. Addition of Conk-S1 significantly reduced the Kᵥ channel-mediated Rb⁺ flux, whereas the K_{ATP} mediated response was unaffected (see Fig. 2a). At 10 µM of Conk-S1 a reduction of ∼25% of the Rb⁺ efflux is observed at all time points while 1 µM significantly inhibited ∼13% of Rb⁺ effluxes at 30 and 40 min. In addition, incubation with Conk-S1 enchanced insulin secretion from the islets (Fig. 2). The observed increase in insulin secretion is small, especially at lower concentrations, but at a relatively high concentrations of Conk-S1 of 10 µM significant for the entire range of glucose concentration tested. Thus, Conk-S1 likely modulates glucose-mediated insulin secretion in pancreatic islets by inhibiting Kᵥ currents mediated by Kᵥ1.7. Furthermore, this effect of Conk-S1 is independent of K_{ATP} activity. The results are in agreement with the idea that blockade of the β-cell delayed rectifier currents would enhance glucose-stimulated insulin secretion.

A decrease in Kᵥ currents of pancreatic cells should modulate membrane potential by delaying cell membrane repolarization. As a result, changes in electrical activity such as action potential frequency and spike amplitude and duration are susceptible to change. Accordingly, the effects of high concentration of Conk-S1 on the electrical activity in the presence of 5 mM and 15 mM glucose were investigated.

The cell in Fig. 3 shows that Conk-S1 generated an increase in firing frequency (∼30%, n=5, Fig. 3A,C) and spike broadening has also been observed (Fig. 3B). Due to the heterogeneity of firing patterns in islet cells quantification of effects was done by determining the total area under the curve with a lower threshold of -75 mV. This analysis revealed and increase in the integrated depolarization (change in voltage × time) of the cells by 28.25 ± 3.5 % (n=5, Fig.3C). Altogether the results demonstrate that the Conk-S1-sensitive Kᵥ current component activity is functionally important for both spike frequency and shaping of the action potentials in β-cells.

Depolarization of β-cells activates L-type calcium channels, leading to an intracellular Ca increase which promotes insulin granule secretion. Therefore, the effects of Conk-S1 on spike frequency and action potential duration should affect the intracellular Ca⁺⁺-concentration of these cells. In Fig. 3D, we show that addition of Conk-S1 generates an increase of about 42±12% in the intracellular Ca⁺⁺ measured with Fluo4 (n=14) in isolated rat islet cells. At 15 mM glucose, Conk-S1 evidenced and increase in the FluoIV signal on cells that showed fast calcium oscillations (n=4, Fig3D upper panel), as well as in cells that were slow or not oscillating (n=10). In 3 separate experiments, we did not see an increase in calcium fluorescence when toxin was added in the presence of 5 mM Glucose, suggesting that Conk-S1 might be delaying cell re-polarization and thereby allowing for higher intracellular calcium accumulation in a glucose-dependent manner, i.e. only during GSIS.

If Conk-S1-specific block of Kᵥ1.7 currents modulates electrical activity and intracellular Ca⁺⁺ in rat pancreatic islet cells, then insulin secretion is expected to be affected *in vivo.* To test this possibility, an oral glucose tolerance test (OGTT) and isulin measurements were performed using healthy SD rats exposed (IV injection) to saline, 100 nmol/Kg Conk-S1 and glibencamide, a K_{ATP} antagonist interacting with sulfonylurea receptor subunits of the channel complex which is used for treatment of type-2 diabetes (Fig. 4A). Conk-S1 application prior to glucose stimulation resulted in a transient increase of insulin release followed by a transient reduction in the glucose-induced blood glucose increase. As expected, glibenclamide also resulted in a reduction of the glucose-induced glucose increase. A major difference between the treatments is that Conk-S1 treatment did not result in glucose reduction; in contrast to glibenclamide, Conk-S1 did not produce hypoglycemia (Fig. 4A). These results are also in agreement with the islet data which show that Conk-S1 does not affect K_{ATP} mediated currents. Despite the fact that Kᵥ1.7 has been reported to be present in skeletal muscle and heart, no obvious side effects of Conk-S1 treatment on the animals during and after the *in vivo* experiments were observed. No animal died during the course of the experiments, and no seizure development was observed. The blood glucose levels of the Conk-S1 treated animals measured one day after the experiments were in the normal range (data not shown).

To explore a possible central nervous system-induced regulation or adaptation during Conk-S1 treatment, glucose was continuously supplied to decerebrated rats and the blood glucose and insulin were measured. Fig. 4B shows that during the "glucose clamp" experiments the glucose-induced increase in blood glucose for control and Conk-S1 treated animals is identical for about the first 15 min of the glucose stimulus. Nevertheless, the steady state level of the blood glucose is significantly reduced for the Conk-S1 treated animals. Consistent with the OGTT experiments no effect on basal glucose levels is observed (Fig. 4A left panel). Interestingly, the insulin release observed during the glucose clamp experiments is transiently increased in, the first 5-10 minutes, in the presence of Conk-S1 (Fig. 4B right panel), but after about 10 min of the glucose stimulus the observed increase in insulin release is almost identical for the control and Conk-S1 treated animals. These results demonstrate that also a constant glucose stimulus is affected by intravenous application of Conk-S1 in that the blood glucose levels are reduced and the initial insulin release is enhanced. The fact that the insulin increase is only initially modulated by Conk-S1 might indicate a transient effect of Conk-S1 that can be down regulated by additional regulatory mechanisms. Since decerebration eliminates central nervous induced regulation or adaptation of the insulin or glucose levels, these results show that the effect of Conk-S1 is due to a peripheral modulation.

### References

Altschul, S.F., Gish, W., Miller, W., Myers, E.W. & Lipman, D.J. (1990) "Basic local alignment search tool." J. Mol. Biol. 215:403-410.
Altschul, S.F. & Gish, W. (1996) "Local alignment statistics." Meth. Enzymol. 266:460-480.
Bax L, Algra A, Mali WP, Edlinger M, Beutler JJ, van der Graaf Y (2008). "Renal function as a risk indicator for cardiovascular events in 3216 patients with manifest arterial disease". Atherosclerosis*.* doi:10.1016/j.atherosclerosis.2007.12.006. PMID 18241872.
Bayrhuber, M., Vijayan, V., Ferber, M., Graf, R., Korukottu, J., Imperial, J., Garrett, J. E., Olivera, B. M., Terlau, H., Zweckstetter, M. and Becker S. Conkunitzin-S1 is the first member of a new Kunitz-type neurotoxin family. J Biol Chem 2005 280(25): 23766-70.
Bayrhuber, M., Graf, R., Ferber, M., Zweckstetter, M., Imperial, J., Garrett, J. E., Olivera, B. M., Terlau, H. and Becker, S. Production of recombinant Conkunitzin-S1 in Escherichia coli. Prot Exp Purif 2006 47: 640-44.
Bebbington, C. R., Renner, G., Thomson, S., King, D., Abrams, D. and Yarranton, G.T. High-level expression of a recombinant antibody from myeloma cells using a glutamine synthetase gene as an amplifiable selectable marker. Biotechnology (1992) 10, 169-175.
Becker, S. and Terlau, H. Toxins from cone snails: properties, applications and biotechnological production. Appl Microbiol Biotechnol 2008.
   Cottingham, I. R., Millar, A., Emslie, E., Colman, A., Schnieke, A. E., and McKee, C. (2001). A method for the amidation of recombinant peptides expressed as intein fusion proteins in Escherichia coli.Nat Biotechnol19: 974-977.
Eberhart, MS; Ogden C, Engelgau M, Cadwell B, Hedley AA, Saydah SH (November 19, 2004). "Prevalence of Overweight and Obesity Among Adults with Diagnosed Diabetes --- United States, 1988--1994 and 1999--2002". Morbidity and Mortality Weekly Report53 (45): 1066-1068. Centers for Disease Control and Prevention. Retrieved on 2007-03-11.
Finol-Urdaneta RK, Strüver N, Terlau H. Molecular and Functional Differences between Heart mKv1.7 Channel Isoforms. J Gen Physiol. 2006 Jul; 128(1):133-45.
Finol-Urdaneta Characterization of human Kv1.7 channel and snp variants Redox influence on human heart surgery patients, *in press*.
Holzgrabe and Bechtold, Deutsche Apotheker Zeitung (2000) 140(8), 813-823.
Jackson, A.C and Bean, B.P. State-dependent enhancement of subthreshold A-type potassium current by 4-aminopyridine in tuberomammillary nucleus neurons. J Neurosci 2007 27(40):10785-10796.
Jacobson DA, Kuznetsov A, Lopez JP, Kash S, Ammälä CE, Philipson LH. Kv2.1 ablation alters glucose-induced islet electrical activity, enhancing insulin secretion. Cell Metab. 2007 Sep;6(3):229-35.
Jacobson DA, Philipson LH. Action potentials and insulin secretion: new insights into the role of Kv channels. Diabetes Obes Metab. 2007 Nov;9 Suppl 2:89-98.
Kubinyi, H. Hausch-Analysis and Related Approaches, VCH Verlag, Weinheim, 1992.
Kalman K, Nguyen A, Tseng-Crank J, Dukes ID, Chandy G, Hustad CM, Copeland NG, Jenkins NA, Mohrenweiser H, Brandriff B, Cahalan M, Gutman GA, Chandy KG. Genomic organization, chromosomal localization, tissue distribution, and biophysical characterization of a novel mammalian Shaker-related voltage-gated potassium channel, Kv1.7. J Biol Chem. 1998 Mar 6;273(10):5851-7.
Kashuba VI, Kvasha SM, Protopopov Al, Gizatullin RZ, Rynditch AV, Wahlestedt C, Wasserman WW, Zabarovsky ER. Initial isolation and analysis of the human Kv1.7 (KCNA7) gene, a member of the voltage-gated potassium channel gene family. Gene. 2001 May 2;268(1-2):115-22.
Liman ER, Tytgat J and Hess P (1992) Subunit Stoichiometry of a Mammalian K+ Channel Determined by Construction of Multimeric cDNAs. Neuron 9:861-871.
Murphy, J.M., Motiwala, R. and Devinsky, O. Phenytoin intoxication. South. Med. J. 1991;84:1199-1204.
Pearson, W. R. and Lipman, D. J. Improved Tools for Biological Sequence Analysis, PNAS (1988) 85:2444-2448.
Pearte CA, Furberg CD, O'Meara ES, et al (2006). "Characteristics and baseline clinical predictors of future fatal versus nonfatal coronary heart disease events in older adults: the Cardiovascular Health Study". Circulation 113 (18): 2177-85. doi:10.1161/CIRCULATIONAHA.105.610352.
Portenoy RK (1989). "Painful polyneuropathy". Neurol Clin 7 (2): 265-88.
Ray, M. V. L, Meenan, C. P., Consalvo, A. P., Smith, C. A., Parton, D. P., Sturmer, A. M., Shields, P. P., and Mehta, N. M. (2002). Production of salmon calcitonin by direct expression of a glycine-extended precursor in Escherichia coli. Protein Expression and Purification 26 (2002) 249-259.
Remedi, M. S., Koster, J. C., Markova, K., Seino, S., Miki, T., Patton, B. L., McDaniel, M. L., and Nichols, C. G. (2004) Diet-Induced Glucose Intolerance in Mice With Decreased {beta}-Cell ATP-Sensitive K+ Channels. Diabetes 53(12), 3159-3167
Remedi MS, R. J., Tong A, Patton BL, McDaniel ML, Piston DW, Koster JC, Nichols CG. (2006) Hyperinsulinism in mice with heterozygous loss of K(ATP) channels. Diabetologia 49(10), 2368-78
Sambrook, Russell "Molecular Cloning, A Laboratory Manual", Cold Spring Harbor Laboratory, N.Y. (2001)
Smith TF, Waterman MS (1981). "Identification of Common Molecular Subsequences". Journal of Molecular Biology 147: 195-197.
Stühmer W. (1992). Electrophysiological recording from Xenopus oocytes. Methods Enzymol. 207:319-39.
Stühmer, W., Terlau, H. and Heinemann, S.H. (1992). Two electrode and patch clamp recordings from Xenopus oocytes In: Practical electrophysiological methods. H. Kettenmann, R. Grantyn (Eds.) Wiley-Liss.
Terlau, H, Boccaccio, A, Olivera, B.M. and Conti, F. The block of Shaker K+ channels by κ-conotoxin PVIIA is state dependent. J Gen Physiol 1999 114:125-140.
Thompson, J.D., Higgins, D.G. and Gibson, T.J. CLUSTAL W: improving the sensitivity of progressive multiple sequence alignment through sequence weighting, position-specific gap penalties and weight matrix choice. Nucleic acids research 1994 22 (22) :4673-80.
Vaillancourt PD, Langevin HM (1999). "Painful peripheral neuropathies". Med. Clin. North Am. 83 (3): 627-42, vi. doi:10.1016/S0025-7125(05)70127-9. PMID 10386118.
Yan L, Figueroa DJ, Austin CP, Liu Y, Bugianesi RM, Slaughter RS, Kaczorowski GJ, Kohler MG. Expression of voltage-gated potassium channels in human and rhesus pancreatic islets. Diabetes. 2004 Mar;53(3):597-607.

## Claims

1. A (poly)peptide or a peptidomimetic thereof having the biological activity of Conkunitzin-S1, wherein said (poly)peptide is selected from
a) a polypeptide comprising or having the amino acid sequence of SEQ ID NO: 1;
b) a polypeptide having at least 85% sequence identity to SEQ ID NO:1; or
c) a fragment of a) or b);
wherein said (poly)peptide or peptidomimetic specifically modulates the activity of a channel having the activity of a Kv1.7 containing channel, for the treatment or prevention of metabolic diseases or conditions, or secondary diseases or conditions related to said metabolic diseases or conditions.

2. A method of treating or preventing metabolic diseases or conditions, or secondary diseases or conditions related to said metabolic diseases or conditions, comprising administering a pharmaceutically effective amount of a (poly)peptide or a peptidomimetic thereof having the biological activity of Conkunitzin-S1, wherein said (poly)peptide is selected from
(a) a polypeptide comprising or having the amino acid sequence of SEQ ID NO: 1;
(b) a polypeptide having at least 85% sequence identity to SEQ ID NO:1; or
(c) a fragment of a) or b);
wherein said (poly)peptide or peptidomimetic specifically modulates the activity of a channel having the activity of a Kv1.7 containing channel, to a subject in need thereof.

3. The (poly)peptide or peptidomimetic of claim 1 or the method of claim 2, wherein said (poly)peptide or peptidomimetic specifically inhibits the activity of a channel having the activity of a Kv1.7 containing channel.

4. The (poly)peptide or peptidomimetic or the method of any one of claims 1 to 3, wherein said metabolic disease or condition is type 2 diabetes, metabolic syndrome, high blood pressure, central obesity, decreased HDL cholesterol or elevated triglycerides.

5. The (poly)peptide or peptidomimetic or the method of any one of claims 1 to 4, wherein said secondary disease or condition related to said metabolic diseases is high blood pressure, diabetic retinopathy, neuropathy, cardiac infarction, peripheral vascular disease, nephropathy, stroke, diabetic foot, venous ulcer, amputation or blindness.

6. A method of screening for (poly)peptides derived from Conkunitzin-S1 suitable for specifically modulating the activity of a channel having the activity of a Kv1.7 containing channel, comprising:
(a) altering the amino acid sequence of Conkunitzin-S1 represented by SEQ ID NO: 1 by deleting and/or inserting and/or replacing at least one amino acid; and
(b) determining the modulatory effect of the (poly)peptide obtained in step (a)
(i) on a channel having the activity of a Kv1.7 containing channel and
(ii) on channels, preferably potassium channels, not having the activity of a Kv1.7 containing channel, which are optionally expressed on the same cell as the channel having the activity of a Kv1.7 containing channel;
wherein a modulatory effect of the (poly)peptide determined in step (i) that is at least 50% ofthe modulatory effect of Conkunitzin-S1 indicates that the (poly)peptide is suitable for modulating the activity of said channel having the activity of a Kv1.7 containing channel; and wherein the determination of essentially no modulatory effect in step (ii) indicates that the (poly)peptide is specifically modulating the activity of channels having the activity of a Kv1.7 containing channel.

7. The method of claim 6, wherein the potassium channels other than those having the activity of a Kv1.7 containing channel are expressed in pancreatic β-cells, cardiac cells, skeletal muscle cells, kidney cells, smooth muscle cells or liver cells.

8. The method of claim 6 or 7, wherein the potassium channels other than those having the activity of a Kv1.7 containing channel are channels consisting of Kv2.1 or 2.2 subunits.

9. The method of any one of claims 6 to 8, wherein said (poly)peptide inhibits the activity of channels having the activity of a Kv1.7 containing channel.

10. The (poly)peptide or peptidomimetic or the method of any one of claims 1 to 9, wherein said channel having the activity of a Kv1.7 containing channel consists of Kv1.7 subunits.

11. The method of any one of claims 6 to 10, further comprising optimizing the pharmacological properties of a (poly)peptide identified as specifically modulating the activity of a channel having the activity of a Kv1.7 containing channel.

12. The method of claim 11, wherein the optimization comprises modifying a peptide identified as specifically modulating the activity of channels having the activity of a Kv1.7 containing channel to achieve:
a) modified site of action, spectrum of activity, organ specificity, and/or
b) improved potency, and/or
c) decreased toxicity (improved therapeutic index), and/or
d) decreased side effects, and/or
e) modified onset of therapeutic action, duration of effect, and/or
f) modified pharmacokinetic parameters (resorption, distribution, metabolism and excretion), and/or
g) modified physico-chemical parameters (solubility, hygroscopicity, color, taste, odor, stability, state), and/or
h) improved general specificity, organ/tissue specificity, and/or
i) optimized application form and route
by
a. esterification of carboxyl groups, or
b. esterification of hydroxyl groups with carboxylic acids, or
c. esterification of hydroxyl groups to, e.g. phosphates, pyrophosphates or sulfates or hemi-succinates, or
d. formation of pharmaceutically acceptable salts, or
e. formation of pharmaceutically acceptable complexes, or
f. synthesis of pharmacologically active polymers, or
g. introduction of hydrophilic moieties, or
h. introduction/exchange of substituents on aromates or side chains, change of substituent pattern, or
i. modification by introduction of isosteric or bioisosteric moieties, or
j. synthesis of homologous compounds, or
k. introduction of branched side chains, or
l. conversion of alkyl substituents to cyclic analogues, or
m. derivatisation of hydroxyl groups to ketales, acetales, or
n. N-acetylation to amides, phenylcarbamates, or
o. synthesis of Mannich bases, imines, or
p. transformation of ketones or aldehydes to Schiff's bases, oximes, acetales, ketales, enolesters, oxazolidines, thiazolidines
q. cyclisation , or
r. allowing post-translational modifications
or combinations thereof.


SEQUENCE LISTING

<110> Max-Planck-Gesellschaft zur Förderung der Wissenschaften
      e.V.; Universität zu Lübeck

<120> Use of Conkunitzin-S1 (Conk-S1) for the modulation of
      glucose-induced insulin secretion

<130> P1751 EP

<160> 2

<170> PatentIn version 3.4

<210> 1
<211> 60
<212> PRT
<213> Conus striatus

<400> 1

Lys Asp Arg Pro Ser Leu Cys Asp Leu Pro Ala Asp Ser Gly Ser Gly
1               5                   10                  15

Thr Lys Ala Glu Lys Arg Ile Tyr Tyr Asn Ser Ala Arg Lys Gln Cys
            20                  25                  30

Leu Arg Phe Asp Tyr Thr Gly Gln Gly Gly Asn Glu Asn Asn Phe Arg
        35                  40                  45

Arg Thr Tyr Asp Cys Gln Arg Thr Cys Leu Tyr Thr
    50                  55                  60

<210> 2
<211> 456
<212> PRT
<213> homo sapiens

<400> 2